# EUROPEAN PATENT SPECIFICATION

(11) **EP 3 367 939 B1**
(45) Date of publication and mention of the grant of the patent: **04.11.2020**
(21) Application number: 16804930.2
(22) Date of filing: 24.10.2016
(51) Int. Cl.: A61B 17/80, A61B 17/82, A61B 17/84, A61B 90/00

(54) **A CABLE SYSTEM WITH SENSOR**
KABELSYSTEM MIT SENSOR
SYSTÈME DE CÂBLE À CAPTEUR

(30) Priority: 26.10.2015 TR 201513361
(43) Date of publication of application: 05.09.2018
(73) Proprietor: Tobb Ekonomi Ve Teknoloji Universitesi, 06520 Cankaya/Ankara (TR)
(72) Inventor: TOLUNAY, Tolga, 06900 Yenimahalle/Ankara (TR); DEMIR, Teyfik, 06520 Cankaya/Ankara (TR); DEMIR, Hilmi Volkan, 06800 Bilkent/Ankara (TR)
(74) Representative: Petraz, Gilberto Luigi
(86) International application number: PCT/TR2016/000146
(87) International publication number: WO 2017/074274

(56) References cited:
- US-A1- 2008 262 549
- US-A1- 2014 243 906

## Description

### Technical Field

This invention is related to a cable system having a sensor, integrated onto the implant, which is attached to the femur bone and which envelopes the femur bone, enabling to measure the force applied on the femur bone by implants providing the required force, and which also carries out the winding force that is required in order for the implant to be used efficiently.

### Prior Art

The hip is one of the largest load bearing joints of our body. The hip joint is formed from the joining of the round end of the thigh bone with the hip bone. The surfaces that come together are coated with cartilage and they obtain their momentum from strong muscles. Different types of prosthesis are used against damages that may occur in said bones, and it is aimed for the femur bone to be strengthened and to be in a state that shall not affect the daily life of a person. However when prosthesis are fixed to the bone, it is difficult to obtain the desired precision, and the bone into which the implant is inserted into can be damaged. If the cable of the supplementary prosthesis having a cable which is one of the prosthesis types is tightened too much, this may damage the bone or may even break or fracture the bone. On the other hand when the cable is not tightened sufficiently, the prosthesis cannot provide its necessary duty and this may necessitate re-operations.

US 2014/0243906 A1 discloses a cable system according to the preamble of claim 1.

### Aims of the Invention

The aim of the invention is to develop a cable system having a sensor, that is attached to the femur bone and which provides the required force by enveloping the femur bone and enables to measure the force applied on the femur bone. Another aim of this invention is to provide a cable system having a sensor which can measure the implant force that can be endured by the femur bone.

Another aim of this invention is to provide a cable system having a sensor which can measure the winding force that is require in order for the implant to be used efficiently.

Another aim of this invention is to provide a cable system having a sensor that can be integrated onto the implant.

The present invention provides a cable system as defined by claim 1. Preferred embodiments are defined by the dependent claims.

### Detailed Description of the Invention

The cable system having a sensor provided in order to reach the aim of the invention has been shown in the attached figures, wherein said figures illustrate the following;

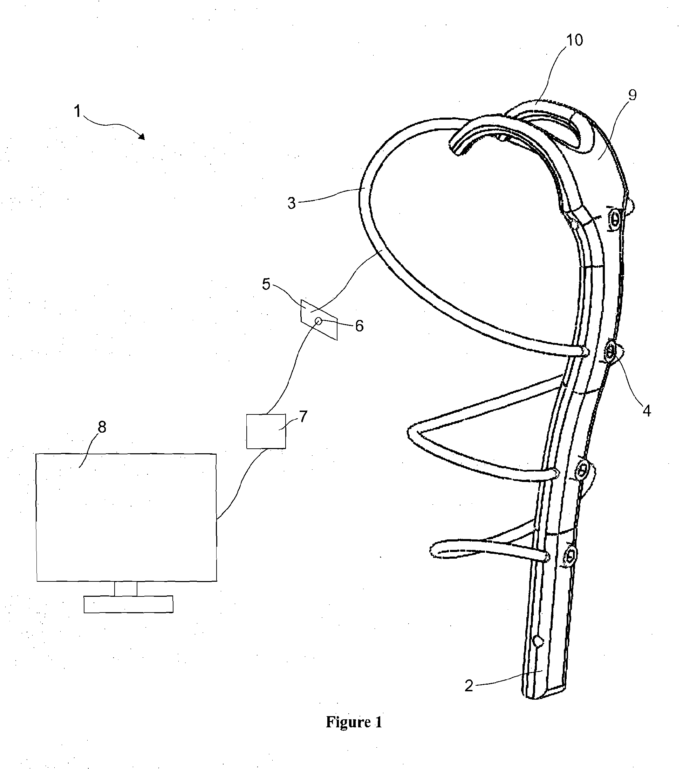

- **Fig. 1.**: Shows the perspective schematic view of the cable system having a sensor.

The parts in the figures are numbered individually and the references corresponding to the related part are listed below.
**1.** Cable system with sensor
**2.** Main Body
**3.** Tightening cable
**4.** Adjustment hole
**5.** Sensor
**6.** Transmitter
**7.** Receiver
**8.** Monitor
**9.** Upper body
**10.** Bone holder

The cable system (1) having a sensor, integrated onto the implant, which is attached to the femur bone and which envelopes the femur bone, enabling to measure the force applied on the femur bone by implants providing the required force, and which also carries out the enveloping force that is required in order for the implant to be used efficiently, basically comprises;
- at least a main body (2),
- At least a tightening cable (3), which extends along the main Body (2) and which enables the implant to envelope the bone,
- At least an adjustment hole (4) located in the main body (2) which enables the winding of the tightening cable (3) around the bone to be wound with force,
- At least a sensor (5) positioned within the tightening cable (3) in order to measure the force applied onto the tightening cable (3), the force direction, displacement, displacement direction, deformation, deformation direction and all other similar data,
- at least a transmitter (6) located within the sensor (5) which transmits the information measured in vivo by the sensor (5) to the outer environment,
- at least a receiver (7) which receives information provided by the transmitter (6),
- at least a monitor (8) which enables to display the information obtained from the receiver (7)
- at least an upper body (9) along the main body (2) which enables the main body (2) to be attached to the bone by means of its concave geometry,
- at least a bone holder (10) located on the main body (9) which enables fitting to the bone,

The cable system (1) with sensor subject to the invention according to the preferred embodiment of the invention comprises a main body (2) and an upper body (9), The tightening cable (3) located on the main body moves along the main body (2) and enables the implant to precisely fit the bone, The adjustment holes (4) located on the main body (2) are aligned preferably along the path from which the tightening cable (3) passes from and by this means the desired level of tightening can be carried out. The tightening cable (3) applies the desired force on the bone by means of the adjustment holes. By this means the bone is both prevented from being damages and it is enabled for the implant to be used more efficiently. The adjustment holes (4) are formed to ve suitable to the required apparatus and the tightening cable (3) is also adjusted with the desired precision by means of said adjustment holes. A sensor (5) is positioned within the tightening cable (3) in order to measure the force applied onto the tightening cable (3), the force direction, displacement, displacement direction, deformation, deformation direction and all other similar data. The desired precision is provided by means of this sensor (5) and the bone is prevented from being damaged. The implant can be fitted into the bone having the desired force by means of the sensor (5) data that is read. A transmitter (6) located on the sensor (5) transmits the information that has been measured to an external source. The transmitter (6) can selectively use the desired protocol and communication structure. A receiver (7) is provided which obtains the information transmitted by the transmitter (6) to the external source. This receiver (7) transmits the information it has received to the monitor (8). The monitor (8) can either show the information or can carry out an analysis process in order to provide the information that is required by the user. Besides these, at least an upper body (9) along the main body (2) which enables the main body (2) to be attached to the bone by means of its concave geometry is provided. The upper body (9) has a structure that can be shaped in accordance with the curve of the bone and by this means it is enabled for the implant to precisely fit the bone. Moreover an exact hold is provided between the bone and the implant by means of the bone holder (10) located along the upper body (9), and therefore the implant is prevented from moving.

In an embodiment of the invention the sensor (5) provided on the cable system (1) with sensor (5) can not only submit the information by means of the transmitter (6) but it can also transmit the information directly.

The force obtained by means of the cable system (1) with a sensor subject to the invention according to another embodiment of the invention, and the force direction, displacement, displacement direction, deformation, deformation direction and all other similar data, can be both viewed and analyzed from the monitor (8). Such information analyzed, can also be supplied in printed form.

## Claims

1. A cable system (1) having an implant and a sensor (5) integrated onto the implant, the implant being configured to be attached to a femur bone and to envelope the femur bone, the sensor (5) enabling to measure a force applied on the femur bone by the implant providing the required force and carrying out the enveloping force that is required in order for the implant to be used efficiently the implant comprising
- at least a main body (2),
- at least a tightening cable (3), which extends along the main body (2) and which enables the implant to envelope the bone,
- at least an adjustment hole (4) located in the main body (2) which enables the winding of the tightening cable (3) around the bone to be wound with force, wherein the sensor (5) is configured to measure the force applied onto the tightening cable (3), the force direction, displacement, displacement direction, deformation, deformation direction and all other similar data,
**characterised in that** the sensor (5) is positioned within the tightening cable (3).

2. A cable system (1) with sensor according to claim 1 **characterized by** at least a transmitter (6) located within the sensor (5) which transmits the information measured in vivo by the sensor (5) to the outer environment.

3. A cable system (1) with sensor according to claim 1 **characterized by** at least a receiver (7) which receives information provided by the transmitter (6).

4. A cable system (1) with sensor according to claim 1 **characterized by** at least a monitor (8) which enables to display the information obtained from the receiver (7)

5. A cable system (1) with sensor according to claim 1 **characterized by** at least an upper body (9) along the main body (2) which enables the main body (2) to be attached to the bone by means of its concave geometry.

6. A cable system (1) with sensor according to claim 1 **characterized by** at least a bone holder (10) located on the main body (9) which enables fitting to the bone.

7. A cable system (1) with sensor according to claim 1 **characterized by** a tightening cable (3) which is located along the main body (2) and which enables the implant to be fitted exactly to the bone.

8. A cable system (1) with sensor according to claim 1 **characterized by** at least a tightening cable (3) which prevents the bone from being damaged and which enables the implant to be used efficiently.

9. A cable system (1) with sensor according to claim 1 **characterized by** an adjustment hole (4) which is preferably placed along the path of the tightening cable (3) thereby enabling a tightening process at the desired level.

10. A cable system (1) with sensor according to claim 1 **characterized by** an adjustment hole (4) which enables the desired amount of force to be applied on the bone by the tightening cable (3) and which enables the tightening cable (3) to be adjusted with precision.

11. A cable system (1) with sensor according to claim 1 **characterized by** a sensor (5) which provides the desired precision and which prevents the bone from being damaged by this means.

12. A cable system (1) with sensor according to claim1 **characterized by** a sensor (5) which enables the implant to be fitted with the desired implant force density to the bone and which can directly transmit information.

13. A cable system (1) with sensor according to claim 1 **characterized by** at least an upper body (9) along the main body (2) which enables the main body (2) to be attached to the bone by means of its concave geometry.

14. A cable system (1) with sensor according to claim 1 **characterized by** a curved upper body (9) which can be shaped and therefore which enables the implant to be fitted exactly to the bone.

15. A cable system (1) with sensor according to claim 1 **characterized by** at least a bone holder (10) located along the main body (9) which enables exact fitting between the implant and the bone.

## Patentansprüche

1. Kabelsystem (1), aufweisend ein Implantat und einen Sensor (5), der auf dem Implantat integriert ist, wobei das Implantat dafür ausgelegt ist, an einem Femurknochen befestigt zu werden und den Femurknochen zu umhüllen, wobei der Sensor (5) ermöglicht, eine Kraft zu messen, die auf den Femurknochen durch das Implantat angewandt wird, das die erforderliche Kraft bereitstellt und die Umhüllungskraft ausübt, die benötigt wird, damit das Implantat wirksam benutzt wird, wobei das Implantat umfasst:
- zumindest einen Hauptkörper (2),
- zumindest ein Spannkabel (3), das sich entlang des Hauptkörpers (2) erstreckt und das dem Implantat ermöglicht, den Knochen zu umhüllen,
- zumindest ein Einstellloch (4), das im Hauptkörper (2) gelegen ist, das das Umwickeln des Spannkabels (3) um den Knochen ermöglicht, damit es mit Kraft umgewickelt wird,
worin der Sensor (5) dafür ausgelegt ist, die Kraft, die auf das Spannkabel (3) angewandt wird, die Kraftrichtung, die Verschiebung, die Verschiebungsrichtung, die Verformung, die Verformungsrichtung und alle anderen ähnlichen Daten zu messen, **dadurch gekennzeichnet, dass** der Sensor (5) innerhalb des Spannkabels (3) positioniert ist.

2. Kabelsystem (1) mit Sensor nach Anspruch 1, **gekennzeichnet durch** zumindest einen Sender (6), der innerhalb des Sensors (5) angeordnet ist, der die vom Sensor (5) *in vivo* gemessenen Informationen der äußeren Umgebung sendet.

3. Kabelsystem (1) mit Sensor nach Anspruch 1, **gekennzeichnet durch** zumindest einen Empfänger (7), der die Informationen empfängt, die vom Sender (6) geliefert werden.

4. Kabelsystem (1) mit Sensor nach Anspruch 1, **gekennzeichnet durch** zumindest einen Bildschirm (8), der ermöglicht, die vom Empfänger (7) erhaltenen Informationen anzuzeigen.

5. Kabelsystem (1) mit Sensor nach Anspruch 1, **gekennzeichnet durch** zumindest einen oberen Körper (9) entlang des Hauptkörpers (2), der dem Hauptkörper (2) ermöglicht, am Knochen durch seine konkave Geometrie befestigt zu werden.

6. Kabelsystem (1) mit Sensor nach Anspruch 1, **gekennzeichnet durch** zumindest einen Knochenhalter (10), der am Hauptkörper (9) gelegen ist, der das Passen zum Knochen ermöglicht.

7. Kabelsystem (1) mit Sensor nach Anspruch 1, **gekennzeichnet durch** ein Spannkabel (3), das entlang des Hauptkörpers (2) gelegen ist und das dem Implantat ermöglicht, zum Knochen genau zu passen.

8. Kabelsystem (1) mit Sensor nach Anspruch 1, **gekennzeichnet durch** zumindest ein Spannkabel (3), das verhindert, dass der Knochen beschädigt wird, und das dem Implantat ermöglicht, wirksam benutzt zu werden.

9. Kabelsystem (1) mit Sensor nach Anspruch 1, **gekennzeichnet durch** ein Einstellloch (4), das vorzugsweise entlang des Pfades des Spannkabels (3) angeordnet ist, womit ein Spannvorgang im gewünschten Maße ermöglicht wird.

10. Kabelsystem (1) mit Sensor nach Anspruch 1, **gekennzeichnet durch** ein Einstellloch (4), das ermöglicht, dass die gewünschte Menge an Kraft auf den Knochen vom Spannkabel (3) angewandt wird, und das ermöglicht, dass das Spannkabel (3) genau eingestellt wird.

11. Kabelsystem (1) mit Sensor nach Anspruch 1, **gekennzeichnet durch** einen Sensor (5), der die gewünschte Genauigkeit liefert und der verhindert, dass der Knochen hierdurch beschädigt wird.

12. Kabelsystem (1) mit Sensor nach Anspruch 1, **gekennzeichnet durch** einen Sensor (5), der ermöglicht, dass das Implantat mit der gewünschten Implantat-Kraftdichte zum Knochen passt, und der Informationen direkt senden kann.

13. Kabelsystem (1) mit Sensor nach Anspruch 1, **gekennzeichnet durch** zumindest einen oberen Körper (9) entlang des Hauptkörpers (2), der dem Hauptkörper (2) ermöglicht, am Knochen durch seine konkave Geometrie befestigt zu werden.

14. Kabelsystem (1) mit Sensor nach Anspruch 1, **gekennzeichnet durch** einen gekrümmten oberen Körper (9), der geformt sein kann, und daher dem Implantat ermöglicht, zum Knochen genau zu passen.

15. Kabelsystem (1) mit Sensor nach Anspruch 1, **gekennzeichnet durch** zumindest einen Knochenhalter (10), der entlang des Hauptkörpers (9) gelegen ist, der das genaue Passen zwischen dem Implantat und dem Knochen ermöglicht.

## Revendications

1. Système de câble (1) ayant un implant et un capteur (5) intégré sur l'implant, l'implant étant configuré pour être fixé à un fémur et pour envelopper le fémur, le capteur (5) permettant de mesurer une force appliquée sur le fémur par : l'implant fournissant la force requise et réalisant la force d'enveloppement qui est requise pour que l'implant soit utilisé efficacement, l'implant comprenant
- au moins un corps principal (2),
- au moins un câble de serrage (3) qui s'étend le long du corps principal (2) et qui permet à l'implant d'envelopper l'os,
- au moins un trou de réglage (4) situé dans le corps principal (2) qui permet d'enrouler avec force le câble de serrage (3) autour de l'os,
où le capteur (5) est configuré pour mesurer la force appliquée sur le câble de serrage (3), la direction de force, le déplacement, la direction de déplacement, la déformation, la direction de déformation et toutes les autres données similaires,
**caractérisé en ce que** le capteur (5) est positionné à l'intérieur du câble de serrage (3).

2. Système de câble (1) avec capteur selon la revendication 1, **caractérisé par** au moins un émetteur (6) situé à l'intérieur du capteur (5) qui transmet les informations mesurées in vivo par le capteur (5) à l'environnement extérieur.

3. Système de câble (1) avec capteur selon la revendication 1, **caractérisé par** au moins un récepteur (7) qui reçoit des informations fournies par l'émetteur (6).

4. Système de câble (1) avec capteur selon la revendication 1, **caractérisé par** au moins un moniteur (8) qui permet d'afficher les informations obtenues à partir du récepteur (7).

5. Système de câble (1) avec capteur selon la revendication 1, **caractérisé par** au moins un corps supérieur (9) le long du corps principal (2) qui permet au corps principal (2) d'être fixé à l'os au moyen de sa géométrie concave.

6. Système de câble (1) avec capteur selon la revendication 1, **caractérisé par** au moins un support osseux (10) situé sur le corps principal (9) qui permet un ajustement à l'os.

7. Système de câble (1) avec capteur selon la revendication 1, **caractérisé par** un câble de serrage (3) qui est situé le long du corps principal (2) et qui permet à l'implant d'être ajusté exactement à l'os.

8. Système de câble (1) avec capteur selon la revendication 1, **caractérisé par** au moins un câble de serrage (3) qui empêche l'os d'être endommagé et qui permet à l'implant d'être utilisé efficacement.

9. Système de câble (1) avec capteur selon la revendication 1, **caractérisé par** un trou de réglage (4) qui est de préférence placé le long de la trajectoire du câble de serrage (3) permettant ainsi un processus de serrage au niveau souhaité.

10. Système de câble (1) avec capteur selon la revendication 1, **caractérisé par** un trou de réglage (4) qui permet d'appliquer la quantité de force souhaitée sur l'os par le câble de serrage (3) et qui permet au câble de serrage (3) d'être réglé avec précision.

11. Système de câble (1) avec capteur selon la revendication 1, **caractérisé par** un capteur (5) qui fournit la précision souhaitée et qui empêche l'os d'être endommagé par ce moyen.

12. Système de câble (1) avec capteur selon la revendication 1, **caractérisé par** un capteur (5) qui permet à l'implant d'être ajusté avec la densité de force d'implant souhaitée à l'os et qui peut transmettre directement des informations.

13. Système de câble (1) avec capteur selon la revendication 1, **caractérisé par** au moins un corps supérieur (9) le long du corps principal (2) qui permet au corps principal (2) d'être fixé à l'os au moyen de sa géométrie concave.

14. Système de câble (1) avec capteur selon la revendication 1, **caractérisé par** un corps supérieur incurvé (9) qui peut être mis en forme et qui permet ainsi à l'implant d'être ajusté exactement à l'os.

15. Système de câble (1) avec capteur selon la revendication 1, **caractérisé par** au moins un support osseux (10) situé le long du corps principal (9) qui permet un ajustement exact entre l'implant et l'os.
